# EUROPEAN PATENT SPECIFICATION

(11) **EP 3 986 572 B1**
(45) Date of publication and mention of the grant of the patent: **19.11.2025**
(21) Application number: 20733447.5
(22) Date of filing: 22.06.2020
(51) Int. Cl.: A61Q 19/00, A61K 8/60, A61K 8/73, A61K 8/99

(54) **COSMETIC COMPOSITION COMPRISING A COMBINATION OF AT LEAST ONE OLIGOSACCHARIDE AND/OR POLYSACCHARIDE COMBINED WITH A MANNOSE MONOSACCHARIDE, AND USE THEREOF IN MAINTAINING THE BALANCE OF THE BACTERIAL SKIN FLORA**
KOSMETISCHE ZUSAMMENSETZUNG MIT EINER KOMBINATION AUS MINDESTENS EINEM OLIGOSACCHARID UND/ODER POLYSACCHARID, KOMBINIERT MIT EINEM MANNOSEMONOSACCHARID SOWIE VERWENDUNG DAVON ZUR BEWAHRUNG DES GLEICHGEWICHTS DER BAKTERIELLEN HAUTFLORA
COMPOSITION COSMÉTIQUE COMPRENANT UNE ASSOCIATION D'AU MOINS UN OLIGOSACCHARIDE ET/OU POLYSACCHARIDE ASSOCIÉ À UN MONOSACCHARIDE MANNOSE ET SON UTILISATION DANS LE MAINTIEN DE L'ÉQUILIBRE DE LA FLORE BACTÉRIENNE CUTANÉE

(30) Priority: 24.06.2019 FR 1906838
(43) Date of publication of application: 27.04.2022
(73) Proprietor: L'OREAL, 75008 Paris (FR)
(72) Inventor: GUENICHE, Audrey, 93601 AULNAY-SOUS-BOIS (FR)
(74) Representative: L'Oreal
(86) International application number: PCT/EP2020/067354
(87) International publication number: WO 2020/260201

(56) References cited:
- EP-A1- 0 591 443
- WO-A1-2018/116238
- WO-A2-2006/134409
- FR-A1- 2 863 887
- FR-A1- 2 916 634
- FR-A1- 3 009 962
- DATABASE GNPD [online] MINTEL; 24 October 2018 (2018-10-24), ANONYMOUS: "Tea Tree Oil & Prebiotic Daily Feminine Wash", XP055667523, retrieved from www.gnpd.com Database accession no. 6073561
- DATABASE WPI Week 201918, Derwent World Patents Index; AN 2019-03725F, XP002797579
- DATABASE GNPD [online] MINTEL; 9 February 2018 (2018-02-09), ANONYMOUS: "D-Mannose Sachets for Urinary Tract Infections", XP055667529, retrieved from www.gnpd.com Database accession no. 5438003
- DATABASE GNPD [online] MINTEL; 4 June 2018 (2018-06-04), ANONYMOUS: "Cell Essence", XP055667569, retrieved from www.gnpd.com Database accession no. 5451883
- DATABASE GNPD [online] MINTEL; 4 July 2018 (2018-07-04), ANONYMOUS: "Cell Essence", XP055667575, retrieved from www.gnpd.com Database accession no. 5807271

## Description

The present invention relates to a cosmetic composition for topical application that comprises in a physiologically acceptable medium a combination of at least one oligosaccharide and/or polysaccharide combined with a mannose monosaccharide. It also relates to uses and methods of cosmetic non-therapeutic treatment that employ this combination in maintaining and and/or restoring the balance of the bacterial flora of the skin and/or mucous membranes.

Human skin is made up of two compartments, namely a deep compartment, the dermis, and a superficial compartment, the epidermis.

The skin has a major role in protection from external aggressions such as environment aggressions, climatic adversities (heat, cold, UV, tobacco), pollution, allergens, pathogenic germs, mechanical aggressions (epilation, shaving, abrasive scrubbing) and chemical aggressions (detergents). This property, called the barrier function, is primarily performed by the outermost layer of the epidermis, namely the horny layer or *stratum corneum.*

The skin also represents a complex ecosystem on which several types of microorganisms, such as bacteria and fungi, proliferate. These microorganisms constitute the skin flora, also called microbial skin flora.

To date, more than 500 bacterial species have been detected on healthy skin, encompassing more than 2 million genes. Around 10⁶ bacteria inhabit each cm² of skin. The microbiome of the skin of healthy subjects has been described as exhibiting specificity according to 3 types of zones: wet, dry and oily.

The following in particular are distinguished:
- the commensal beneficial resident flora consists of microorganisms proliferating conventionally and permanently on healthy skin drawing their nutrients from the skin, and providing known benefits to the skin; a particular representative is the strain *Staphylococcus epidermidis.* This strain is found in particular on the face on healthy skin, where it participates in maintaining the balance of the commensal skin flora.
- **the transitory** flora, present on the skin under abnormal conditions, for example by contact with soiled elements; this flora may become pathogenic if it proliferates. A possible example is *Staphylococcus aureus,* which is potentially pathogenic for human skin.

In cases where the barrier is adversely affected or when the balance between commensals and pathogenic agents is disturbed, for example subsequent to an external aggression, a deterioration in the quality of the skin and/or skin disorders may result from this, such as, for example, instances of irritation, stinging, pulling, redness, itching, or even skin disorders such as atopic dermatitis, acne, or skin infections (Functions of the skin microbiota in health and disease. James A. Sanford, Richard L. Gallo. Seminars in Immunology 25 (2013) 370-377; The human skin microbiome. Allyson L. Byrd, Yasmine Belkaid and Julia A. Segre. Nature Reviews Microbiology, 16, 143-155, 2018).

It is apparent, moreover, that the quality of the skin barrier and of the mucous membranes is affected daily due to external aggressions from irritants (detergents, surfactants, acids, bases, oxidizing agents, reducing agents, concentrated solvents), mechanical stresses (rubbing, scrubbing, shaving or epilation) or thermal or climatic imbalances (cold, dryness).

Accordingly, the (re)colonization of the skin by beneficial microorganisms of the commensal skin flora appears to be fundamental to the physiology and immunity of the skin, especially by virtue of their effects on ^{[1]-[9]}:
- limitation of the adhesion of pathogenic microorganisms (by stimulating antimicrobial peptides or by competitive adhesion);
- modulation of inflammation and immunity;
- cicatrization;
- restoration/recovery of the barrier function, especially following an external aggression as stated above.

There are already cosmetic solutions known for acting on the microbial skin flora.

US20180325968 describes, in particular, a product comprising a composition of prebiotic lipids and a composition containing probiotics such as *Bacillus licheniformis, Bifidobacterium breve, Bifidobacterium infantis, Lactobacillus fermentum, Lactobacillus plantarum, Lactobacillus rhamnosus, Lactobacillus sakei, Lactobacillus paracasei, Staphylococcus epidermidis,* and *Staphylococcus xylosus,* for correcting a dysbiosis (colonization of pathogenic microorganisms) or for maintaining the balance of the resident beneficial commensal flora.

Furthermore, FR3064473 discloses the use of pullulan or a derivative thereof or combination thereof with a polysaccharide selected from hyaluronic acid or one of its salts or derivatives, alginic acid, one of its salts or derivatives, and a mixture of these polysaccharides, for maintaining and/or preserving the balance of the microbial skin and/or mucosal flora.

CN107412045 discloses a prebiotic skincare mask comprising, in particular, alpha glucooligosaccharide, inulin, a yeast, and a bacterium of the genus *Lactobacillus,* for reinforcing the presence of a healthy skin flora and preventing the colonization of the skin by pathogens.

EP591443 discusses cosmetics that foster the growth of beneficial endogenous flora, guiding the metabolism of the entire endogenous microflora to maintain the skin and mucous membranes' physicochemical balance without promoting the development of pathogenic microorganisms, especially it discloses a face cream formulation that includes GOS. However, this document does not disclose the presence of fructo-oligosaccharides (FOS) and mannose.

FR2916634 focuses on developing a cosmetic composition that activates the skin's saprophytic flora, thereby effectively balancing its surface ecosystem, especially it discloses a skincare cream, a lotion, and a cream for treating oily skin, all three of which contain FOS. However, this document, does not disclose the presence of GOS and mannose.

Nevertheless, the ever-increasing need among consumers for treatment respectful of their skin and especially of their skin flora is leading to a search for new compounds which are active in maintaining or restoring the balance of the bacterial skin and/or mucosal flora.

Accordingly, a need exists to provide new ingredients which are active on the microbial skin flora, particularly by protecting the commensal flora, and which enable the maintenance and/or restoration of the balance of the bacterial flora of the skin and/or mucous membranes.

Unexpectedly, the inventors have found that the composition according to claim 1 has the capacity to maintain and/or restore the balance of the microbial skin and/or mucosal flora, in particular by restoring the appropriate quantity of bacteria on the skin (i.e. bacterial load), and also the diversity of the bacteria on the skin (i.e. alpha and beta diversities), these two parameters being especially decreased in the event of external aggressions.

Moreover, this composition enables prevention and/or treatment of detractions from the aesthetic qualities of the skin and/or mucous membranes, especially prevention and/or treatment of dry and/or rough skin.

"Detraction from the aesthetic qualities of the skin" refers to all of the modifications to the skin that detract from its aesthetic appearance, in particular the appearance of a rough texture and/or the presence of signs of skin dryness, preferably of the facial skin, in particular the cheeks and/or the forehead.

Moreover, this composition enables the prevention and/or non-therapeutic treatment of sensations of discomfort of sensitive skin chosen from itching, heating, or sensations of stinging, and/or of pulling.

A subject of the present invention is therefore a cosmetic composition according to claim 1.

In particular, said oligosaccharide and/or polysaccharide and said mannose monosaccharide are prebiotics.

The present invention also concerns a non-therapeutic method of cosmetic treatment for caring for the skin and/or mucous membranes, which comprises the application to the skin and/or the mucous membranes, in particular having undergone external aggression, of the composition according to the invention.

In particular, the non-therapeutic method of cosmetic treatment according to the invention makes it possible to maintain and/or restore the balance of the bacterial flora of the skin and/or mucous membranes.

Moreover, the non-therapeutic method of cosmetic treatment according to the invention makes it possible to prevent and/or treat detractions from the aesthetic qualities of the skin and/or mucous membranes, especially to prevent and/or treat dry and/or rough skin.

The non-therapeutic method of cosmetic treatment according to the invention also makes it possible to prevent and/or treat sensations of discomfort of sensitive skin chosen from itching, heating, or sensations of stinging, and/or of pulling.

The present invention also relates to the topical cosmetic non-therapeutic use of said combination, and optionally of at least one probiotic microorganism, for caring for the skin, in particular skin having undergone external aggression.

The invention is also directed to the non-therapeutic use of said combination for maintaining and/or restoring the balance of the bacterial flora of the skin and/or mucous membranes.

Furthermore, the invention relates to the non-therapeutic use of said combination for preventing and/or treating sensations of discomfort of sensitive skin chosen from itching, heating, or sensations of stinging, and/or of pulling.

Lastly, the invention concerns the non-therapeutic use of said combination for preventing and/or treating detractions from the aesthetic qualities of the skin and/or mucous membranes, especially for preventing and/or treating dry and/or rough skin.

### Definitions

The term "prebiotics" refers to a substrate which is used selectively by a host microorganism to produce an advantage for health [Gibson, Glenn R.; Hutkins, Robert; Sanders, Mary Ellen; Prescott, Susan L.; Reimer, Raylene A.; Salminen, Seppo J.; Scott, Karen; Stanton, Catherine; Swanson, Kelly S.; Cani, Patrice D.; Verbeke, Kristin; Reid, Gregor (2017). "Expert consensus document: The International Scientific Association for Probiotics and Prebiotics (ISAPP) consensus statement on the definition and scope of prebiotics". Nature Reviews Gastroenterology & Hepatology. 14 (8): 491-502.].

Prebiotics are used especially to maintain an environment which is favourable to the development of the beneficial endogenous skin flora. Prebiotics are used in particular for orienting the metabolism of the entirety of the skin flora so as to maintain a good balance of said flora while not promoting the development of certain pathogenic bacteria. For this purpose, the prebiotics employed in the context of the present invention are those which can be metabolized by the beneficial bacteria of the skin flora. According to one particularly preferred embodiment, the prebiotic used in the compositions of the invention is a prebiotic which is metabolized by the beneficial germs of the skin but which is not metabolizable by the undesirable germs responsible for the skin disorders whose cosmetic treatment is desired. According to this embodiment, the prebiotics modulate the composition and/or the activity of the natural ecosystem, by promoting those microorganisms which exert a positive effect on the skin.

Oligosaccharides and polysaccharides are carbohydrates.

The oligosaccharides and/or polysaccharides can be produced in particular from glucose, galactose, xylose, maltose, sucrose, lactose, starch, xylan, hemicellulose, inulin, gums, in particular from acacia gum, or a mixture thereof.

The term "oligosaccharides" is intended to mean oligoholosides or alternatively oligosides, which are oligomers made up of a number n of monosaccharides via one or more alpha or beta glycosidic bonds, the number n being between 2 and 10, preferably between 2 and 6. Mention may in particular be made of fructooligosaccharides, glucooligosaccharides, oligosaccharides derived from soya, pyrodextrins, isomaltooligosaccharides, xylooligosaccharides and transgalactooligosaccharides.

The term "polysaccharide" is intended to mean polyosides, polyholosides, or else complex carbohydrates which are polymers consisting of several units n of monosaccharides linked together by saccharide bonds, the number n being greater than or equal to 11, preferably between 11 and 200, even better still between 11 and 100, even more preferably between 20 and 80. Mention may especially be made of inulin.

The expression "composition comprising a physiologically acceptable medium" is intended to mean a composition comprising a medium compatible with the skin.

According to one particular embodiment, the pH of the cosmetic composition is between 4 and 7.5, notably between 4.5 and 7, and in particular between 4.7 and 6.

The term "skin" is intended to mean all the skin of the human body, and preferably the skin of the face, the skin of the scalp, neckline, neck, arms and forearms, or even, more preferably, the skin of the face, notably of the forehead, nose, cheeks, chin and area around the eyes.

According to the invention, the term "preventing" or "prevention" is intended to mean reducing the risk of occurrence or slowing down the occurrence of a given phenomenon.

The term "treating" or "treatment" is intended to mean any action that aims to improve the comfort or the well-being of an individual; this term therefore covers attenuating, relieving as well as curing.

In the sense of the present invention, the term "external aggression" refers to environmental aggressions, climatic aggressions (heat, cold, UV, tobacco), pollution, allergens, pathogenic germs, mechanical adversities (epilation, shaving, abrasive scrubbing), and chemical aggressions (detergents).

The term "bacterial load" refers to the quantity of bacteria, in particular the quantity of bacterial DNA found on the skin and/or the mucous membranes.

The term "relative abundance" refers to the proportion of each genus/bacterium in a sample or a population under study.

The term "alpha diversity" or "Shannon index" refers to the number of different bacterial species/genera in a sample or a population under study; the greater the number of different bacterial species/genera, the more rich and diversified the sample or the population.

In particular, the Shannon index is calculated for each sample or population under study and is based on three different parameters:
- the number of different bacterial species/genera;
- the incidence of the bacterium in question in the sample or population under study, in particular whether the bacterium is present in all the individuals of a sample or population or only in a small number of individuals;

- the weight of the each bacterial species/genus identified in the sample or population under study, the calculation taking account of the relative abundance defined above.

The term "beta diversity" refers to the number of different bacterial species/genera between two samples or populations, in other words the number of bacterial species/genera which two samples or populations do not have in common. Conversely to the alpha diversity, therefore, the beta diversity is calculated on the basis of two samples or populations.

The beta diversity is based on three different parameters (such as the UniFrac distance):
- the number of different bacterial species/genera;
- their relative abundance (i.e. the weight of each bacterial species/genus);
- the phylogenetic distinction between these bacterial species/genera.

### Detailed description of the invention

### Oligosaccharide, Polysaccharide

The composition according to the disclosure comprises at least one oligosaccharide and/or polysaccharide selected from the group consisting of inulins, fructooligosaccharides, glucooligosaccharides, soya-derived oligosaccharides, pyrodextrins, isomaltooligosaccharides, xylooligosaccharides, transgalactooligosaccharides and mixtures thereof.

By way of example, mention will be made of the following oligosaccharides and/or polysaccharides.

### Inulin

Inulin is particularly abundant in the rhizomes of plants, in particular the Jerusalem artichoke and chicory from which it is extracted industrially. It is also found in other plants belonging to the family Asteraceae such as Jerusalem artichokes or dahlia onions and burdock. It is considered a soluble dietary fibre.

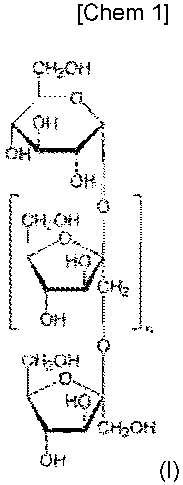

Inulins are polydispersed linear polymers of general formula (I): GFn (G = glucose, F = fructose, n ranging from 2 to more than 60), the fructose units being linked together by a β (2 → 1) bond. Inulins therefore correspond to a chain of fructose units terminated by a glucose unit.

Among the inulins which can be used and which are commercially available, mention may be made of Inutec H25P, n of between 2 and 7, and Inutec N25 from Orafti (average n = 25).

### Fructooligosaccharides

Fructooligosaccharides (or FOSs), also called oligofructoses or oligofructans, are short chains of fructose linked together by β-1,2 bonds.

The fructooligosaccharides (or FOSs) correspond to general formula (II): G(F)ₙ where G is a glucose unit, F is fructose unit and n ranges from 1 to 10.

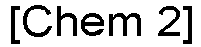

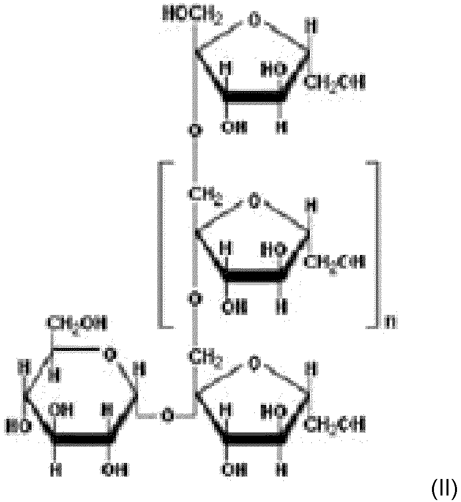

Fructooligosaccharides (FOSs) are produced:
- by partial enzymatic hydrolysis of inulin (e.g.: Raftilose^{®} from Orafti-Belgique), or
- by enzymatic synthesis from sucrose (e.g.: Actilight^{®} from Beghin Meiiji industries-France), or
- by extraction from yacon or jicama (*Polymnia sonchifolia,* synonym: *Smallanthus sonchifolia*); in particular, the extraction is carried out in the absence of solvent, by cold pressing of yacon tubers.

The FOS used in the invention can be a mixture of FOSs. Mention may in particular be made of GF2 + GF3 + GF4 mixtures such as Quantom FOS95 from Quantum hi-Tech or Actilight^{®} from Beghin Meiiji industries-France, the latter corresponding to a mixture of 37% GF2, 53% GF3 and 10% GF4.

### Glucooligosaccharides GOSs

Glucooligosaccharides (GOSs), or oligoglucans, are oligosaccharides constituted of a sequence of α-1,6-linked glucose units which can also contain α-1,2; α-1,3; α-1,4 bonds. They are synthesized by a transglucosylation reaction catalyzed by enzymes of the glucan-sucrase family.

Preferably, the glucooligosaccharides are oligosaccharides constituted of a sequence of α-1,6- and α-1,2-linked glucose units.

Advantageously, the number of glucose units is between 2 and 10, even better still between 4 and 6, even more preferably the number of glucose units is 4.

In addition, the glucooligosaccharides can be synthesized by the polymerization of glucose molecules, a reaction catalyzed by a specific enzyme of the glycosyltransferase type, extracted and purified from a bacterial strain of Leuconostoc mesenteroides. This reaction requires the use of an acceptor: maltose (Glucose-Glucose) but also of a glucose donor: sucrose (Glucose-Fructose).

In a preferred embodiment, the glucooligosaccharides (GOSs) have the formula (III) below:

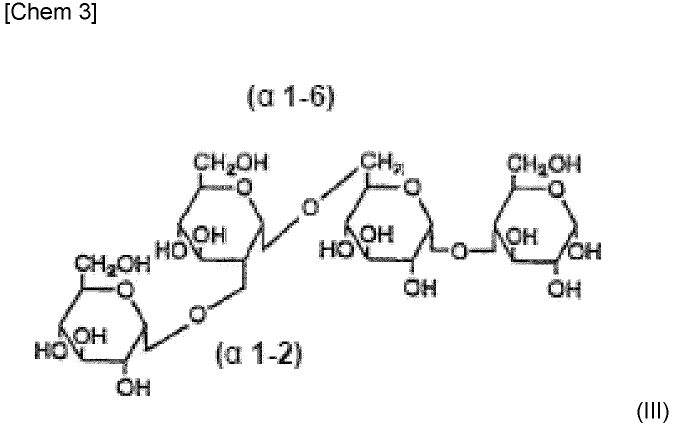

Among the GOSs which can be used and which are commercially available, mention may be made of Bioecolia^{®} from Solabia.

### Soya-derived oligosaccharides

They are extracted directly from the soya bean and do not require any enzymatic treatment. They naturally contain raffinose and stachyose, the formula of which is [α-D-Gal-(1→6)-]ₘ-α-D-Glu-(1→2)-β-D-Fru, with m=1 for raffinose and m=2 for stachyose.

The derived oligosaccharides which can be used include Soya-oligo from Calpis Food Ind. Japan.

### Pyrodextrins

Pyrodextrins are a mixture of oligosaccharides from the hydrolysis of starch.

### Isomaltooligosaccharide

They are produced from starch. These are α-(1,6)-linked glucose oligomers with a degree of polymerization of between 2 and 5. By way of example, Isomalto900P from Showa Sango may be used.

### Xylooligosaccharide

Xylooligosaccharides are oligosaccharides consisting of β-(1,4)-linked xylose. By way of example, Xylo 95P from Suntory Limited may be used.

### Transgalactooligosaccharide

These are linear oligomers of galactose, of chemical structure α-D-glucose-(1→4)-[β-D-galactose-(1→6)-]n (2 <n <5) obtained by fermentation of lactose. By way of example, TOS 100 from Yakult Honsha Co. Ltd. Japan may be used.

The oligosaccharide(s) and/or polysaccharide(s) according to the invention is (are) present in the composition according to the invention (i.e. the total concentration of oligosaccharide(s) and/or polysaccharide(s) in the composition according to the invention) in a concentration of between 0.01% and 20% by weight, preferably between 0.05% and 10% by weight, even better still between 0.05% and 5% by weight relative to the total weight of the composition.

### Mixture of oligosaccharides and/or polysaccharides

According to the invention, the oligosaccharide(s) and/or polysaccharide(s) are used in the composition according to the invention in the form of a mixture.

According to the disclosure, it may be a mixture of oligosaccharides of the same type. For example, as mentioned above, a mixture of FOSs may be used, in particular a mixture of GF2+GF3+GF4, such as Quantom FOS95 from Quantum hi-Tech or Actilight^{®} from Beghin Meiiji industries-France, the latter corresponding to a mixture of 37% GF2, 53% GF3 and 10% GF4.

According to the invention, it is a mixture of oligosaccharides and/or polysaccharides of different types. The disclosure relates in particular to the use of a mixture of inulin with a GOS, an FOS, soya-derived oligosaccharides, pyrodextrins, an isomaltooligosaccharide, a xylooligosaccharide and/or a transgalactooligosaccharide. According to the disclosure, a mixture of inulin and a GOS is used, such as Bioline from Gova Ingredients. The invention relates to the use of a mixture of a GOS with an FOS.

According to the invention, the composition comprises a mixture of oligosaccharides comprising:
- at least one glucooligosaccharide (GOS), and
- at least one fructooligosaccharide (FOS),

wherein said glucooligosaccharide (GOS) is present in the composition according to the invention in a concentration of between 0.01% and 10% by weight, preferably between 0.05% and 5% by weight, even better still between 0.1% and 1%, even more preferably between 0.2% and 0.8% by weight relative to the total weight of the composition;
wherein said fructooligosaccharide (FOS) is present in the composition according to the invention in a concentration of between 0.001% and 5% by weight, preferably between 0.01% and 1% by weight, even better still between 0.01% and 0.5%, even more preferably between 0.05% and 0.3% by weight relative to the total weight of the composition.

Advantageously, the composition according to the invention comprises a mixture of oligosaccharides comprising:
- at least one glucooligosaccharide (GOS), and
- at least one fructooligosaccharide (FOS),
in which the [GOS/FOS] mass ratio is at least 2, preferably is between 2 and 4, even better still between 3 and 4.

Use may in particular be made of the mixture of prebiotic oligosaccharides and probiotic microorganisms sold under the name Ecoskin^{®} by Solabia.

Said mixture comprises in particular:
- between 60% and 80% by weight of at least one glucooligosaccharide (GOS) relative to the total weight of the mixture, and
- between 10% and 25% by weight of at least one fructooligosaccharide (FOS) relative to the total weight of the mixture.

In particular, said mixture comprises 70% by weight of glucooligosaccharide (GOS), 19% by weight of *Polymnia sonchifolia* tuber juice, 1% by weight of *Lactobacillus acidophilus* and *Lactobacillus casei,* 10% by weight of maltodextrin.

### Probiotics

Independently of the abovementioned two variants, the composition according to the invention can also comprise at least one probiotic microorganism.

For the purposes of the present invention, the term "probiotic microorganism" is intended to mean a live microorganism which, when consumed in adequate amount, has a positive effect on the health of its host ("Joint FAO/WHO Expert Consultation on Evaluation of Health and Nutritional Properties of Probiotic in Food Including Powder Milk with Live Lactic Acid Bacteria, 6 October 2001"), and which may in particular improve the intestinal microbial balance.

In the case of the skin, said probiotic microorganism is a probiotic microorganism which, when applied to the skin in an appropriate amount, has a positive effect on the aesthetic qualities of the skin and mucous membranes.

More particularly, they are probiotic microorganisms from the group of lactic acid bacteria, such as in particular *Lactobacillus.* By way of illustration of these lactic acid bacteria, mention may more particularly be made of *Lactobacillus casei, Lactobacillus acidophilus* and mixtures thereof.

Specific examples of probiotic microorganisms are *Lactobacillus acidophilus, Lactobacillus alimentarius, Lactobacillus curvatus, Lactobacillus delbruckii subsp. Lactis, Lactobacillus gasseri, Lactobacillus johnsonii, Lactobacillus reuteri, Lactobacillus casei, Lactobacillus rhamnosus (Lactobacillus* GG), *Lactobacillus sake, Lactococcus lactis, Streptococcus thermophilus, Lactobacillus acidophilus, L. delbrueckii, L. helveticus, L. salivarius, L. curvatus, L. plantarum, L. sakei, L. brevis, L. buchneri, L. fermentum, L. reuteri, Lactobacillus bulgaricus, Lactobacillus longum, Lactobacillus lactis, Bifidobacterium longum* and mixtures thereof.

In general, the compositions according to the invention generally comprise from 0.0001 to 20% by weight of at least one probiotic microorganism relative to the total weight of the composition.

Advantageously, the probiotic microorganism is present in the composition according to the invention in a concentration of between 0.0001% and 10% by weight, preferably between 0.001% and 5% by weight, even more preferably between 0.001% and 1% by weight relative to the total weight of the composition.

The microorganism(s) may be included in a composition according to the invention in a live, semi-active or inactivated, dead form.

They may also be included in the form of fractions of cell components or in the form of metabolites. The microorganism(s), metabolite(s) or fraction(s) may also be introduced in the form of a lyophilized powder, a culture supernatant and/or, where appropriate, in a concentrated form.

According to one preferred embodiment of the invention, these microorganisms are in inactivated form, inactivated in particular by heat or by high pressure.

The terms "in inactivated form", "in non-revivable form" and "in dead form" are synonymous here.

Bacteria "in semi-active form" are bacteria which have partially or totally lost their proliferation properties.

In the case where the microorganisms are formulated in a composition in a live form, the amount of live microorganisms may range from 10³ to 10¹⁵ cfu/g, in particular from 10⁵ to 10¹⁵cfu/g and more particularly from 10⁷ to 10¹²cfu/g of microorganisms per gram of composition.

In one particularly preferred embodiment, the composition according to the invention comprises a mixture of oligosaccharides comprising:
- at least one glucooligosaccharide (GOS), and
- at least one fructooligosaccharide (FOS),
   wherein said glucooligosaccharide (GOS) is present in the composition according to the invention in a concentration of between 0.01% and 10% by weight, preferably between 0.05% and 5% by weight, even better still between 0.1% and 1%, even more preferably between 0.2% and 0.8% by weight relative to the total weight of the composition;
   wherein said fructooligosaccharide (FOS) is present in the composition according to the invention in a concentration of between 0.001% and 5% by weight, preferably between 0.01% and 1% by weight, even better still between 0.01% and 0.5%, even more preferably between 0.05% and 0.3% by weight relative to the total weight of the composition;
and in addition at least one probiotic microorganism chosen from bacteria of the *Lactobacillus* genus, in particular *Lactobacillus casei, Lactobacillus acidophilus* and mixtures thereof, wherein said probiotic microorganism is between 0.0001% and 10% by weight, preferably between 0.001% and 5% by weight, even better still between 0.001% and 1% by weight relative to the total weight of the composition.

Advantageously, the composition according to the invention comprises a mixture of oligosaccharides comprising:
- at least one glucooligosaccharide (GOS), and
- at least one fructooligosaccharide (FOS),
in which the [GOS/FOS] mass ratio is at least 2, preferably is between 2 and 4, even better still between 3 and 4.

Use may in particular be made of the mixture of prebiotic oligosaccharides and probiotic microorganisms sold under the name Ecoskin^{®} by Solabia.

Said mixture comprises in particular:
- between 60% and 80% by weight of at least one glucooligosaccharide (GOS) relative to the total weight of the mixture, and
- between 10% and 25% by weight of at least one fructooligosaccharide (FOS) relative to the total weight of the mixture, and
- between 0.001% and 15% by weight of at least one probiotic microorganism, in particular chosen from *Lactobacillus casei, Lactobacillus acidophilus* and mixtures thereof, relative to the total weight of the mixture.
In particular, said mixture comprises 70% by weight of glucooligosaccharide (GOS), 19% by weight of *Polymnia sonchifolia* tuber juice, 1% by weight of *Lactobacillus acidophilus* and *Lactobacillus casei,* 10% by weight of maltodextrin.

### Mannose

The composition according to the invention comprises a mannose monosaccharide.

Mannose is a monosaccharide (simple non-hydrolysable sugar) consisting of 6 carbons; it is a hexose. Its empirical formula is C₆H₁₂O₆, the same as glucose, of which it is the C₂ epimer (that is to say that its spatial configuration is strictly the same, except for the substituent on carbon 2, where it is reversed compared to glucose).

The monosaccharide in question according to the invention corresponds to formula (IV) below.

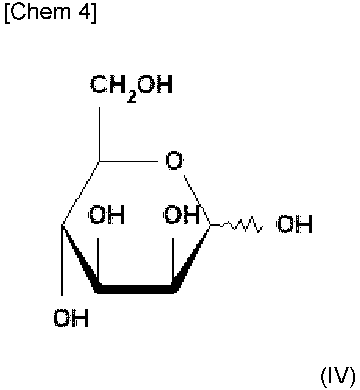

Of course, all of the enantiomeric forms of mannose are considered according to the invention.

The mannose may also be in solvated form (including hydrates) and in the form of a mixture of D and L stereoisomers: DL-mannose.

According to one preferred embodiment, the mannose monosaccharide is the D-mannose of formula (V) below.

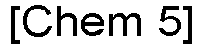

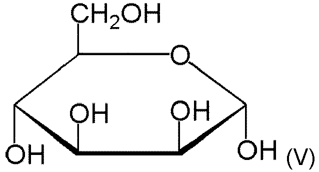

D-Mannose is naturally present in plants, in particular certain fruits, including cranberries, or in hardwood (beech and birch).

As an illustration of D-mannose suitable for the invention, mention may in particular be made of D-mannose sold by the company Danisco Sweeteners^{®} or else the company Symrise^{®}.

In general, the mannose monosaccharide can be used from 0.01% to 20% by weight, preferably from 0.05% to 10% by weight, and most preferably from 0.1% to 5% by weight, relative to the total weight of the composition containing it.

As mentioned previously, the composition according to the invention comprises a physiologically acceptable medium. More particularly, said physiologically acceptable medium may comprise water and/or one or more water-soluble organic solvents which can be chosen from linear or branched C₁-C₆ monoalcohols such as ethanol, isopropanol, tert-butanol or n-butanol; polyols such as glycerol, propylene glycol, hexylene glycol (or 2-methyl-2,4-pentanediol), and polyethylene glycols; polyol ethers such as dipropylene glycol monomethyl ether; and mixtures thereof.

Preferably, the composition according to the invention has a water content ranging from 20% to 95% by weight, even better still from 40% to 90% by weight relative to the total weight of the composition.

Advantageously, the composition comprises one or more water-soluble organic solvents in a content ranging from 0.5% to 25% by weight, preferably from 5% to 20% by weight, even better still from 10% to 15% by weight relative to the total weight of the composition.

### Presentation forms

A cosmetic composition according to the invention is applied topically.

The compositions intended for external topical administration may be aqueous, aqueous-alcoholic or oily solutions, solutions or dispersions of the lotion or serum type, emulsions of liquid or semi-liquid consistency of the milk type, obtained by dispersion of a fatty phase in an aqueous phase (O/W) or vice versa (W/O), or suspensions or emulsions, of soft, semi-solid or solid consistency, of the cream type, aqueous or anhydrous gels, microemulsions, microcapsules, microparticles, or vesicular dispersions of ionic and/or non-ionic type.

These compositions are prepared according to the usual methods.

A topical composition according to the invention can advantageously be formulated in any presentation form suitable for skin care, in particular in the form of creams for cleansing, protecting, treating or caring for the face, or for the body, of a mask to be left placed on the skin or the hair, of makeup-removal milks, of body protection or care milks, of lotions, gels or foams for skin care, such as cleansing lotions, bath compositions.

Alternatively, a topical composition according to the invention can advantageously be formulated in any presentation form suitable for hair care, in particular in the form of a hair lotion, of a shampoo, in particular an anti-dandruff, of a conditioner, of a disentangler, a hair cream or gel, of a treating lotion, of a lotion or gel for preventing hair loss, of an antiparasitic shampoo, or of a treating shampoo, in particular an anti-seborrhoeic shampoo, of a scalp care, in particular anti-irritant, anti-ageing, restructuring, product.

### Adjuvants

In a known manner, presentation forms intended for topical administration may also contain adjuvants that are common in the cosmetic, pharmaceutical and/or dermatological field, such as hydrophilic or lipophilic gelling agents, hydrophilic or lipophilic active agents, preservatives, antioxidants, solvents, fragrances, fillers, screening agents, fatty substances such as oils, emulsifiers, odour absorbers and colourants. The amounts of these various adjuvants are those conventionally used in the field under consideration, for example from 0.01% to 20% of the total weight of the composition. Depending on their nature, these adjuvants may be introduced into the fatty phase and/or into the aqueous phase.

As hydrophilic gelling agents that can be used in the composition according to the invention, mention may be made of modified or unmodified carboxyvinyl polymers, such as the products sold under the names Carbopol (INCI name: carbomer) and Pemulen (INCI name: Acrylates/C10-30 alkyl acrylate crosspolymer) by the company Goodrich, or such as the crosslinked sodium polyacrylate sold under the name Cosmedia SP by the company Cognis (INCI name: Sodium polyacrylate); polyacrylamides; 2-acrylamido-2-methylpropanesulfonic acid homopolymers such as the product sold by Clariant under the name Hostacerin AMPS (INCI name: ammonium polyacryldimethyltaurate); crosslinked anionic copolymers of acrylamide and AMPS, provided in the form of an emulsion, such as those sold under the name of Sepigel 305 (CTFA name : Polyacrylamide/C13-14 isoparaffin/Laureth-7) and under the name Simulgel 600 (CTFA name : Acrylamide/Sodium acryloyldimethyltaurate copolymer/ Isohexadecane/Polysorbate 80) by the company SEPPIC; acrylate/acrylonitrile copolymers such as Hypan SS201 sold by the company Kingston; synthetic neutral polymers such as poly-N-vinylpyrrolidone; polysaccharides such as guar gum, xanthan gum and cellulose-based derivatives. The amount of these polymers can range, for example, from 0.05% to 5% by weight and better still from 0.1% to 3% by weight relative to the total weight of the composition.

Lipophilic gelling agents that may be mentioned include modified clays, such as bentones, metal salts of fatty acids, such as aluminium stearates, and hydrophobic silica, or alternatively ethylcellulose and polyethylene.

When a composition of the invention is an emulsion, the proportion of the fatty phase may range from 5% to 80% by weight and preferably from 10% to 50% by weight relative to the total weight of the composition. The oils, emulsifiers and coemulsifiers used in the composition in emulsion form are chosen from those conventionally used in cosmetics and/or dermatology. The emulsifier and the coemulsifier may be present in the composition in a proportion ranging from 0.3% to 30% by weight and preferably from 0.5% to 20% by weight relative to the total weight of the composition.

When the composition of the invention is an oily solution or gel, the fatty phase may represent more than 90% of the total weight of the composition.

As oils that may be used, examples that may be mentioned include:
- hydrocarbon-based oils of plant origin, such as liquid fatty acid triglycerides including from 4 to 10 carbon atoms, for instance heptanoic or octanoic acid triglycerides, or alternatively, for example, sunflower oil, corn oil, soybean oil, marrow oil, grapeseed oil, sesame seed oil, hazelnut oil, apricot oil, macadamia oil, arara oil, castor oil, avocado oil, caprylic/capric acid triglycerides, for instance those sold by the company Stearineries Dubois or those sold under the names Miglyol 810, 812 and 818 by the company Dynamit Nobel, jojoba oil and shea butter oil;
- synthetic esters and ethers, especially of fatty acids, for instance the oils of formulae R₁COOR₂ and R₁OR₂ in which R₁ represents a fatty acid residue containing from 8 to 29 carbon atoms and R₂ represents a branched or unbranched hydrocarbon-based chain containing from 3 to 30 carbon atoms, for instance purcellin oil, isononyl isononanoate, isopropyl myristate, 2-ethylhexyl palmitate, 2-octyldodecyl stearate, 2-octyldodecyl erucate or isostearyl isostearate; hydroxylated esters, for instance isostearyl lactate, octyl hydroxystearate, octyldodecyl hydroxystearate, diisostearyl malate, triisocetyl citrate, and fatty alcohol heptanoates, octanoates and decanoates; polyol esters, for instance propylene glycol dioctanoate, neopentyl glycol diheptanoate and diethylene glycol diisononanoate; and pentaerythritol esters, for instance pentaerythrityl tetraisostearate;
- linear or branched hydrocarbons, of mineral or synthetic origin, such as liquid paraffins, which may be volatile or non-volatile, and derivatives thereof, petroleum jelly, polydecenes, hydrogenated polyisobutene such as parleam oil, fatty alcohols having from 8 to 26 carbon atoms, such as cetyl alcohol, stearyl alcohol and the mixture thereof (cetylstearyl alcohol), octyldodecanol, 2-butyloctanol, 10 2-hexyldecanol, 2-undecylpentadecanol, oleyl alcohol or linoleyl alcohol; silicone oils such as volatile or non-volatile polymethylsiloxanes (PDMSs) with a linear or cyclic silicone chain, which are liquid or pasty at ambient temperature, in particular cyclopolydimethylsiloxanes (cyclomethicones) such as cyclohexasiloxane; polydimethylsiloxanes comprising alkyl, alkoxy or phenyl groups, which are pendent or at the end of the silicone chain, said groups having from 2 to 24 carbon atoms; phenylated silicones such as phenyl trimethicones, phenyl dimethicones, phenyltrimethylsiloxydiphenylsiloxanes, diphenyl dimethicones, diphenylmethyldiphenyltrisiloxanes, 2-phenylethyltrimethylsiloxysilicates, and polymethylphenylsiloxanes; and
- mixtures thereof.

In the list of oils mentioned above, the term "hydrocarbon-based oil" means any oil predominantly including carbon and hydrogen atoms, and possibly ester, ether, fluoro, carboxylic acid and/or alcohol groups.

As emulsifiers, mention may be made of amphoteric, cationic, anionic or non-ionic surfactants, used alone or in a mixture, and optionally a co-emulsifier.

For O/W emulsions, mention may for example be made, as emulsifiers, of non-ionic emulsifiers such as oxyalkylenated (more particularly polyoxyethylenated) fatty acid esters of glycerol; oxyalkylenated fatty acid esters of sorbitan; oxyalkylenated (oxyethylenated and/or oxypropylenated) fatty acid esters; oxyalkylenated (oxyethylenated and/or oxypropylenated) fatty alcohol ethers; sugar esters, in particular oxyalkylenated sugar esters, esters of phosphoric acid and of a fatty alcohol; and mixtures thereof.

In one preferred embodiment, the composition according to the invention also comprises at least one ingredient chosen from silicone fatty substances such as silicone oils, gums and waxes; non-silicone fatty substances such as oils, pastes and waxes of plant, mineral, animal and/or synthetic origin; fatty acids having from 8 to 32 carbon atoms; synthetic esters and ethers, in particular of formula R₁COOR₂ and R¹OR² in which R¹ represents the residue of a fatty acid comprising from 8 to 29 carbon atoms, and R² represents a branched or unbranched hydrocarbon-based chain containing from 3 to 30 carbon atoms; linear or branched hydrocarbons of mineral or synthetic origin; fatty alcohols having from 8 to 26 carbon atoms; water; C2-C6 monoalcohols; glycols chosen from propylene glycol, butylene glycol, pentylene glycol; ketones; thickeners, emulsifiers, surfactants, gelling agents, fragrances, fillers, colourants, moisturizers, vitamins chosen from vitamin A, E, B3, polymers.

The amounts of these various ingredients are those conventionally used in the field under consideration, for example from 0.01% to 20% of the total weight of the composition.

### Cosmetic treatment methods and cosmetic uses

The present invention also concerns a non-therapeutic method of cosmetic care treatment of the skin and/or mucous membranes, which comprises the application to the skin and/or the mucous membranes, in particular having undergone external aggression, of the composition according to the invention.

In particular, the non-therapeutic method of cosmetic treatment according to the invention makes it possible to maintain and/or restore the balance of the bacterial flora of the skin and/or mucous membranes.

Moreover, the non-therapeutic method of cosmetic treatment according to the invention makes it possible to prevent and/or treat detractions from the aesthetic qualities of the skin and/or mucous membranes, especially to prevent and/or treat dry and/or rough skin.

The non-therapeutic method of cosmetic treatment according to the invention also makes it possible to prevent and/or treat sensations of discomfort of sensitive skin chosen from itching, heating, or sensations of stinging, and/or of pulling.

The present invention also relates to the topical cosmetic non-therapeutic use of said combination as defined above, and optionally of at least one probiotic microorganism as defined above, for caring for the skin, in particular skin having undergone external aggression.

The invention is also directed to the non-therapeutic use of said combination for maintaining and/or restoring the balance of the bacterial flora of the skin and/or mucous membranes.

Furthermore, the invention relates to the non-therapeutic use of said combination for preventing and/or treating sensations of discomfort of sensitive skin chosen from itching, heating, or sensations of stinging, and/or of pulling.

According to the invention, as regards the discomfort sensations are itching, heating, or sensations of stinging, and/or of pulling. According to the disclosure, representative visible cutaneous signs that may especially be mentioned include pruritus, dry patches, erythema and/or redness.

These phenomena are more common in the most exposed areas of the body, namely the hands, the feet, the face and the scalp.

Thus, the appearance of the signs of discomfort, which appear within minutes of the individual coming into contact with the triggering factor, is one of the essential characteristics of sensitive skin. Those primarily concerned are dysaesthesic sensations, which are neurosensory signs. Dysaesthesic sensations are understood to be sensations such as stinging, tingling, itching, heating, discomfort, pulling. These subjective signs usually exist in the absence of visible clinical signs such as desquamations. It is nowadays known that these cutaneous intolerance reactions are especially associated with a release of neuropeptides by the nerve endings of the epidermis and the dermis.

The present invention is additionally directed to preventing and/or treating the manifestations of dysaesthetic sensations in sensitive skin.

The disclosure also proposes to prevent and/or reduce sensations of skin discomfort, such as, for example, stinging, tingling, itching, heating, discomfort, erythema and/or pulling, in particular in people with sensitive skin.

The invention also proposes to prevent and/or reduce sensations of skin discomfort, chosen from itching, heating, or sensations of stinging, and/or of pulling.

In contrast to skin qualified as being "allergic", the reactivity of sensitive skin is not an immunological process, i.e. it is not produced in response to the presence of an allergen.

Furthermore, its response mechanism is said to be "non-specific". In this regard, it is distinct from skin which manifests inflammatory and allergic reactions such as dermatosis, eczema and/or ichthyosis.

For the purposes of the present invention, the term "sensitive skin" covers irritable skin and intolerant skin.

In the context of the present invention, the sensitive skin under consideration does not show any inflammatory nature.

Lastly, the invention relates to the non-therapeutic use of said combination for preventing and/or
treating detractions from the aesthetic qualities of the skin and/or mucous membranes, especially for preventing and/or treating dry and/or rough skin.

Dry skin manifests itself essentially in a sensation of discomfort, such as sensations of pulling and/or tautness. This dry skin is also rough to the touch and/or appears to be covered with squamae. When the skin is slightly dry, the squamae are abundant but barely visible to the naked eye. They become less and less numerous, but increasingly visible to the naked eye, when this disorder worsens.

The cause of this skin dryness may be of constitutional or acquired type.

Said combination is aimed preferably at treating non-pathological constitutional dry skin or non-pathological acquired dry skin.

In the case of acquired dry skin, the involvement of external parameters such as exposure to chemical agents, to inclement climatic conditions or the sun, or else certain therapeutic treatments (with retinoids, for example) is a determining factor. Under these external influences, the skin may then become momentarily and locally dry.

Non-pathological constitutional dry skin is dry skin the severity of which may be dependent on external factors as already indicated. Included in this skin category are senile skin (characterized by a general decrease in skin metabolism with age), fragile skin (very sensitive to external factors and often accompanied by erythema) and common xerosis (of probable genetic origin and manifesting itself mainly on the face, the limbs and the back of the hands).

The examples that follow illustrate the invention, and are given purely as nonlimiting illustrations.

Throughout the text which follows, the percentages are given on a weight basis, unless otherwise mentioned.

The expression "at least one" is equivalent to "one or more".

The expressions "between... and..." and "ranging from... to...", "at least..." or "at most" should be understood as being limits inclusive, unless otherwise specified.

The examples and figures that follow are provided as illustrations which do not limit the field of the invention.

### Examples

### Example 1 - Evaluation of the recovery of bacterial skin flora following application of a composition according to the invention after use of an aggressive cleanser.

A group of 30 women between 30 and 50 years old was selected. An aggressive cleanser was used to carry out the cleansing of the skin of the face of the subjects in the study.

Ingredients of the composition of the cleanser included the following: Water, triethanolamine, glycerin, myristic acid, lauric acid, cocamidopropyl betaine, lauryl phosphate, hydroxypropyl methylcellulose, pentasodium triphosphate, O-cymen-5-ol (isopropyl methylphenol), benzophenone-4, butyl hydroxy toluene.

The skin of the cheeks of the subjects was evaluated:
- on bare skin (D0): at T0be, T0af, T3h and T6h;
- immediately after application of composition 1 (D5): at T3h and T6h;
- after 14 days of treatment with composition 1 (D19): at T3h and T6h.

T0 be: before cleansing;
T0 af: immediately after cleansing;
   T3h: 3 h after cleansing;
   T6h: 6 h after cleansing.
Evaluation is carried out on the following criteria:
   roughness:
      - roughness was evaluated by clinical score: the clinician evaluated the roughness of the skin to the touch, without touching the area of the cheek on which sampling had been carried out;
      - the scale is from 0 to 4 as follows:
         0 = absent: surface perfectly smooth and supple
         1 = slight: slight irregularity and roughness on tangential touching
         2 = moderate: marked irregularity, rough appearance and slight induration of the skin, detected by vertical touching
         3 = severe: more pronounced irregularity and roughness, combined with induration of the skin
         4 = extreme: very marked irregularity and major disturbance of skin marking with more marked signs.
   dryness:
      - dryness was evaluated by clinical score: the clinician studied the dryness of the skin of the face.
      - the scale is from 0 to 3 as follows:
         0 = skin not dry
         1 = slight dryness (slight roughness)
         2 = moderate dryness (moderate roughness, a certain desquamation)
         3 = severe dryness (marked roughness and marked scaling).
   sensations of discomfort:
      - sensations of discomfort (stinging, itching, heat, pulling) were evaluated by self-evaluation: the interrogator asked the volunteer to carry out self-evaluation just after intensive cleansing and during kinetics after 3 h and 6 h, using the following scale from 0 to 4:
         0 = not at all
         1 = slightly
         2 = moderately
         3 = severe
         4 = very severe.

Analysis of bacterial load and bacterial communities:
- the total bacterial load was evaluated by QPCR (QPCR on V1-V3 16S rRNA in triplicate (primer set 27F/534R) KAPA Biosystems kit with SYBER Green n)
- a metagenomic analysis of the bacterial communities was carried out with the aid of new-generation sequencing (amplification of V1-V3 (primer set 27F/534R) on the Illumina MiSeq platform) and then the taxonomic assignments were made using the SILVA base, and the OTUs were analyzed by QIIME (open reference), thus making it possible to calculate the alpha diversity (Shannon index) and the beta diversity (UniFrac distance):

The following composition 1 according to the invention was prepared:

**[Table 1]**

| **Composition 1 according to the invention** | |
|---|---|
| INCl names | Concentrations (m/m) |
| CAPRYLOYL GLYCINE | 0.5 |
| **MANNOSE*** | **0.5** |
| **ALPHA-GLUCAN OLIGOSACCHARIDE (and) POLYMNIA SONCHIFOLIA tuber (juice) (and) LACTOBACILLUS**** | **1** |
| MYRISTYL MYRISTATE | 1 |
| CYCLOHEXASILOXANE | 5 |
| XANTHAN GUM | 0.1 |
| HYDROGENATED POLYISOBUTENE | 5 |
| AMMONIUM POLYACRYLOYLDIMETHYLTAURATE | 1.5 |
| GLYCERYL STEARATE SE | 0.5 |
| POTASSIUM CETYL PHOSPHATE | 0.5 |
| STEARIC ACID | 0.5 |
| ACRYLONITRILE/METHYL METHACRYLATE/VINYLIDENE CHLORIDE COPOLYMER | 0.3 |
| GLYCERIN | 5 |
| CAPRYLYL GLYCOL | 0.25 |
| SODIUM HYDROXIDE | 0.096 |
| WATER | qs 100 |

| | |
|---|---|
| *D-Mannose, sold by the company Danisco. ** Ecoskin^{®} sold by the company Solabia comprising 70% by weight of glucooligosaccharide (GOS), 19% by weight of Polymnia sonchifolia tuber juice, 1% by weight of Lactobacillus acidophilus and Lactobacillus casei, 10% by weight of maltodextrin. | |

Composition 1 is prepared as follows: the components of the aqueous phase are homogenized with stirring and the whole is heated to a temperature of 60°C. Added to this heated mixture are the components of the fatty phase, which beforehand have been premixed and heated to a temperature of 60°C. The gelling agents are added subsequently. The whole is kept under stirring to emulsification, and the mixture is cooled to a temperature of 22°C to 23°C with continued stirring.

Composition 1 was applied to the entirety of the face.

### Evaluation of skin roughness

**[Table 2]**

| | D0 | D5 | D19 |
|---|---|---|---|
| T0 before cleansing | 0 | 0 | 0 |
| T0 after cleansing | 1.600 (σ 0.119) | 1.600 (σ 0.119) | 1.428 (σ 0.119) |
| | | Application of composition 1 | |
| T3h after cleansing | 1.324 (σ: 0.119) | 1.083 (σ 0.119) | 0.945 (σ 0.119) |
| T6h after cleansing | 1.186 (σ 0.119) | 0.876 (σ 0.119) | 0.531 0.119) |

The values set out in table 2 above represent the means (n=30) of the skin roughness score, evaluated according to the scale detailed above.

Conclusion: using the area under the curve, after fourteen days of treatment (at D19), application of composition 1 according to the invention shows a significant decrease in skin roughness relative to non-application of the composition (p<0.001).

### Evaluation of skin dryness

**[Table 3]**

| | D0 | D5 | D19 |
|---|---|---|---|
| T0 before cleansing | 0 | 0 | 0 |
| T0 after cleansing | 1.25 (σ 0.09) | 1.25 (σ 0.09) | 1.112 (σ 0.09) |

| | | Application of composition 1 | |
|---|---|---|---|
| T3h after cleansing | 1.043 (σ 0.09) | 0.802 (σ 0.09) | 0.353 (σ 0.09) |
| T6h after cleansing | 0.974 (σ 0.09) | 0.733 (σ 0.09) | 0.147 0.09) |

The values set out in table 3 above represent the means (n=30) of the skin dryness score, evaluated according to the scale detailed above.

### Conclusion:

- using the area under the curve, after fourteen days of treatment (at D19), application of composition 1 according to the invention shows a significant decrease in skin dryness relative to non-application of the composition (p=0.0001),
- after fourteen days of treatment (at D19), application of composition 1 according to the invention shows a significant decrease in skin dryness relative to a single application of the composition at D5 (p<0.0001).

### Evaluation of discomfort sensations

**[Table 4]**

| | D0 | D5 | D19 |
|---|---|---|---|
| T0 before cleansing | 0 | 0 | 0 |
| T0 after cleansing | 3.00 (σ 0.168) | 2.586 (σ 0.168) | 2.241 (σ 0.168) |

| | | Application of composition 1 | |
|---|---|---|---|
| T3h after cleansing | 2.69 (σ 0.168) | 1.724 (σ 0.168) | 0.966 (σ 0.168) |
| T6h after cleansing | 1.724 (σ 0.168) | 1.103 (σ 0.168) | 0.621 0.168) |

The values reported in table 4 above represent the means (n=30) of the skin discomfort sensation score, evaluated according to the scale detailed above.

### Conclusion:

- using the area under the curve, application of composition 1 according to the invention shows a significant decrease in the skin discomfort sensations after a single application at D5, relative to the non-application of the composition (p<0.001).
- also, after fourteen days of treatment (at D19), application of composition 1 according to the invention shows a significant decrease in the skin discomfort sensations relative to a single application of the composition at D5 (p<0.001).
- lastly, after fourteen days of treatment (at D19), application of composition 1 according to the invention shows a significant decrease in the skin discomfort sensations relative to the non-application of the composition (p<0.001).

### Evaluation of the log base 10 bacterial load

**[Table 5]**

| | D0 | D5 | D19 |
|---|---|---|---|
| T0 before cleansing | 5.2 (σ 0.115) | 5.182 (σ 0.115) | 5.2 (σ 0.115) |
| T0 after cleansing | 4.899 (σ 0.115) | 4.734 (σ 0.115) | 4.75 (σ 0.115) |

| | | Application of composition 1 | |
|---|---|---|---|
| T3h after cleansing | 4.614 (σ 0.115) | 5.113 (σ 0.115) | 4.966 (σ 0.115) |
| T6h after cleansing | 4.84 (σ 0.115) | 5.2 (σ 0.115) | 5.2 (σ 0.115) |

The values reported in table 5 above represent the means (n=30) for bacterial load using the evaluation method detailed above.

### Conclusion:

- using the area under the curve, application of composition 1 according to the invention shows a significant increase in the bacterial load after a single application at D5 relative to the non-application of the composition (p<0.001).
- also, after fourteen days (at D19), application of composition 1 according to the invention shows a significant increase in the bacterial load relative to the non-application of the composition (p<0.001), indicating complete recovery of the bacterial load.

### Evaluation of alpha diversity (Shannon index)

**[Table 6]**

| | D0 | D5 | D19 |
|---|---|---|---|
| T0 before cleansing | 1.800 (σ 0.09) | 1.900 (σ 0.09) | 2.00 (σ 0.09) |
| T0 after cleansing | 1.112 (σ 0.09) | 1.100 (σ 0.09) | 1.144 (σ 0.09) |

| | | Application of composition 1 | |
|---|---|---|---|
| T3h after cleansing | 1.223 (σ 0.09) | 0.908 (σ 0.09) | 1.319 (σ 0.09) |
| T6h after cleansing | 1.31 (σ 0.09) | 1.426 (σ 0.09) | 1.570 0.09) |

The values reported in table 6 above represent the median alpha diversities using the evaluation method detailed above.

### Conclusion

- a significant decrease in alpha diversity is found after cleansing (p<0.05). No recovery is found on bare skin, with a significant decrease (p<0.05) in the diversity still present at 3 h and 6 h after cleansing.
- starting from initial application of composition 1 according to the invention at D5, the beginning of recovery of the alpha diversity (p=0.024) is observed, heralding an onset of recovery.
- after fourteen days (at D19), application of composition 1 according to the invention shows a significant increase in the alpha diversity, indicating a recovery of the alpha diversity, with a diversity at T6h after cleansing no different from T0 before cleansing (p>0.05), this being an indicator of complete recovery of the alpha diversity.
- also, after fourteen days (at D19), application of composition 1 according to the invention shows a significantly different recovery of the alpha diversity relative to the non-application of the composition (p=0.023).

Evaluation of beta diversity / UniFrac distance - value R representing the phylogenetic distance relative to T0 before cleansing.

**[Table 7]**

| | D0 | D5 | D19 |
|---|---|---|---|
| T0 after cleansing | R = 0.156 | R = 0.244 | R = 0.131 |
| | P = 0.0075 | P = 0.0075 | P = 0.0075 |

| | | Application of composition 1 | |
|---|---|---|---|
| T3h after cleansing | R = 0.262 | R = 0.127 | R = 0.092 |
| | P = 0.0075 | P = 0.03 | P = 000729 |
| T6h after cleansing | R = 0.184 | R = 0.12 | R = 0.094 |
| | P = 00018 | P = 0.03 | P = 000729 |

The values reported in table 7 above represent the means (n=30) of the beta diversity using the evaluation method detailed above. The greater the reduction in the value R, the lower the phylogenetic distance relative to T0 before cleansing, and the greater the recovery capacity of the bacterial community.

### Conclusion:

- cleansing with the aggressive cleanser brings about a considerable modification in the structure of the bacterial communities of the skin. It is important to note that the change after T3h is greater than at T0 after cleansing, meaning that the skin bacteria have difficulties in recovery.
- a lower R value is already observed after just a single application of composition 1 according to the invention, relative to non-treatment, and this signifies the start of recovery.
- fourteen days of applying composition 1 according to the invention lead to a lesser alteration just after cleansing and also to an R value that is still lower relative to a single application: 3 h after cleansing, the value of R is very low and the value p is no longer significantly different, suggesting that the bacterial community is similar to that before cleansing, and indicating total recovery of the bacterial beta diversity.

### Example 2 - Comparison of the recovery in bacterial skin flora following application, between a composition according to the invention and a non-inventive composition, after use of an aggressive cleanser.

A group of 30 women between 55-65 years old was selected. An aggressive cleanser was used to carry out the cleansing of the skin of the face of the subjects in the study. The protocol applied was the same as that set out in example 1 above, but with composition 1 being replaced by compositions 2 (according to the invention) and 3 (non-inventive).

Ingredients of the composition of the cleanser included the following: Water, triethanolamine, glycerin, myristic acid, lauric acid, cocamidopropyl betaine, lauryl phosphate, hydroxypropyl methylcellulose, pentasodium triphosphate, O-cymen-5-ol (isopropyl methylphenol), benzophenone-4, butyl hydroxy toluene.
The skin of the cheeks of the subjects was evaluated:
- on bare skin (D0): at T0be, T0af, T3h and T6h;
- immediately after application of the composition (D3): at T3h and T6h;
- after 15 days of treatment with the composition (D18): at T3h and T6h.

T0 be: before cleansing;
T0 af: immediately after cleansing;
T3h: 3 h after cleansing;
T6h: 6 h after cleansing.
Evaluation is carried out on the following criteria:
   - pulling sensations
   - bacterial load
   - alpha diversity (Shannon index)
   - beta diversity

The scoring scales used are the same as those indicated in example 1.

The following composition 2 according to the invention and non-inventive composition 3 were prepared:

**[Table 8]**

| INCI names | **Composition 2 according to the invention (m/m)** | **Composition 3 outside of the invention (m/m)** |
|---|---|---|
| LYSATE OF BIFIDOBACTERIUM LONGUM | 10 | 10 |
| 95% ETHANOL | 5 | 5 |
| **MANNOSE*** | **0.5** | - |
| ACTIVE AGENTS | 1.755 | 1.755 |
| SODIUM HYDROXIDE | 0.0345 | 0.0345 |
| **ALPHA-GLUCAN OLIGOSACCHARIDE (and) POLYMNIA SONCHIFOLIA tuber juice (and) LACTOBACILLUS**** | **0.3** | - |
| SACCHAROMYCES CEREVISIAE YEAST EXTRACT | **1** | 1 |
| ROSA GALLICA FLOWER EXTRACT | 0.01 | - |
| OIL | 1.5 | 1.5 |
| FRAGRANCE | qs | qs |
| GELLING AGENT | 0.35 | 0.35 |
| OXYETHYLENATED (60 EO) HYDROGENATED CASTOR OIL | 0.0165 | 0.0165 |
| PEG-20 METHYL GLUCOSE SESQUISTEARATE | 0.1 | 0.1 |
| DISODIUM EDTA | 0.1 | 0.1 |
| GLYCOL | 5 | 5 |
| PRESERVATIVES | qs | qs |
| WATER | qs 100 | qs 100 |

| | | |
|---|---|---|
| *D-Mannose, sold by the company Danisco. *** Ecoskin^{®} sold by the company Solabia comprising 70% by weight of glucooligosaccharide (GOS), 19% by weight of Polymnia sonchifolia tuber juice, 1% by weight of Lactobacillus acidophilus and Lactobacillus casei, 10% by weight of maltodextrin.* | | |

Compositions 2 and 3 were prepared as follows: the compounds cannot be dissolved cold and are therefore mixed and heated to 80°C to form a pre-emulsion. Compositions 2 and 3 are then cooled to 35°C. The gelling agents are subsequently added and dissolved. The probiotic fractions are added at ambient temperature.

Compositions 2 and 3 were applied to the entirety of the face.

### Evaluation of pulling sensations

**[Table 9]**

| | D0 | D3 | D18 |
|---|---|---|---|
| T0 before cleansing | 0 | 0 | 0 |
| T0 after cleansing | 2.467 (σ 0.507) | 1.967 (σ 0.850 | 1.500 (σ 0.630) |

| | | Application of composition 3 outside of the invention | |
|---|---|---|---|
| T3h after cleansing | 1.767 (σ 0.679) | 0.533 (σ 0.629) | 0.367 (σ 0.669) |
| T6h after cleansing | 1.400 (σ 0.563) | 0.233 (σ 0.728) | 0.267 (σ 0.691) |

**[Table 10]**

| | D0 | D3 | D18 |
|---|---|---|---|
| T0 before cleansing | 0 | 0 | 0 |
| T0 after cleansina | 2.636 (σ 0.489) | 2.061 (σ 0.747) | 1.606 (σ 0.556) |

| | | Application of composition 2 according to the invention | |
|---|---|---|---|
| T3h after cleansing | 1.606 (σ 0.659) | 0.697 (σ 0.728) | 0.455 (σ 0.506) |
| T6h after cleansing | 1.242 (σ 0.502) | 0.030 (σ 0.951) | 0.30 0.174) |

Conclusion: from the first application of the two compositions 2 and 3, relative to bare skin (D0), a significantly more rapid decrease (p<0.001) is observed in instances of pulling induced by the aggressive cleanser. After 15 days of application of the two compositions 2 and 3, they provide significant protection against the pulling sensations induced by the aggressive cleanser, relative to bare skin (p<0.001).

### Evaluation of bacterial load

**[Table 11]**

| | D0 | D3 | D18 |
|---|---|---|---|
| T0 before cleansing | 5.400 (σ 0.507) | 4.900 (σ 0.500) | 5.000 (σ 0.600) |
| T0 after cleansing | 5.170 (σ 0.725) | 4.712 (σ 0.536) | 4.815 (σ 0.619) |

| | | Application of composition 3 outside of the invention | |
|---|---|---|---|
| T3h after cleansing | 4.963 (σ 0.680) | 5.461 (σ 0.581) | 5.345 (σ 0.642) |
| T6h after cleansing | 5.094 (σ 0.694) | 5.453 (σ 0.492) | 0.501 (σ 0.560) |

**[Table 12]**

| | D0 | D3 | D18 |
|---|---|---|---|
| T0 before cleansing | 5.400 (σ 0.500) | 5.300 (σ 0.499) | 5.500 (σ 0.489) |
| T0 after cleansina | 5.214 (σ 0.516) | 5.167 (σ 0.129) | 5.335 (σ 0.616) |

| | | Application of composition 2 according to the invention | |
|---|---|---|---|
| T3h after cleansing | 5.165 (σ 0.517) | 5.996 (σ 0.457) | 5.966 (σ 0.570) |
| T6h after cleansing | 5.384 (σ 0.471) | 6.000 (σ 0.477) | 6.103 (σ 0.491) |

Conclusion: application of the two compositions 2 and 3 allows the bacterial load to be recovered significantly more rapidly and completely from 3 h (p<0.0001); this efficacy is also confirmed after 15 days of application, where the two formulas allow significantly complete and more rapid recovery of the total bacterial load (p<0.0001) from 3 h.

### Evaluation of alpha diversity (Shannon index)

**[Table 13]**

| | D0 | D3 | D18 |
|---|---|---|---|
| T0 before cleansing | 4.777402 (σ 1.889741) | 4.908551 (σ 1.714782) | 4.886903 (σ 1.851608) |
| T0 after cleansing | 3.463015 (σ 1.720453) | 3.296553 (σ 1.466964) | 3.137147 (σ 1.374128) |

| | | Application of composition 3 outside of the invention | |
|---|---|---|---|
| T3h after cleansing | 3.379283 (σ 1.348742) | 3.599526 (σ 1.557088) | 3.848689 (σ 1.383819) |
| T6h after cleansing | 3.516270 (σ 1.618340) | 3.792114 (σ 1.477205) | 3.899070 (σ: 1.552283) |

The values reported in table 13 (composition 3) above represent the means of alpha diversity evaluated by the method detailed above.

The recovery observed at D3 is better, but not significantly different relative to D0, and no significant difference is observed in recovery of the bacterial diversity between D0 (bare skin), D3 (one application) and D18 (14 days of application). There is therefore no better recovery by comparison with bare skin.

**[Table 14]**

| | D0 | D3 | D18 |
|---|---|---|---|
| T0 before cleansing | 4.672790 (σ 1.855959) | 4.873973 (σ 1.999028) | 4.884979 (σ 2.051535) |
| T0 after cleansing | 3.051956 (σ 1.559591) | 3.453342 (σ 1.598937) | 3.124363 (σ 1.434146) |

| | | Application of composition 2 according to the invention | |
|---|---|---|---|
| T3h after cleansing | 3.333296 (σ 1.465144) | 3.781791 (σ 1.539461) | 3.754071 (σ 1.693631) |
| T6h after cleansing | 3.426750 (σ 1.587532) | 3.793559 (σ 1.615621) | 4.106017 (σ 1.809540) |

The values reported in table 14 (composition 2 according to invention) above represent the means of alpha diversity evaluated by the method detailed above.

A significant recovery is observed at D18 (T6h) relative to D0 (T6h) (p<0.005). After 15 days of use, the bacterial skin diversity is recovered 2.73 times faster after an aggression. The recovery is greater and more rapid at D18 relative to bare skin at D0 and also to a single application of composition 2 at D3.

Evaluation of beta diversity - value R representing the phylogenetic distance relative to T0 before cleansing.

**[Table 15]**

| | D0 | D3 | D18 |
|---|---|---|---|
| T0 after cleansing | R = 0.057 | R = 0.097 | R = 0.160 |
| | P = 0.0880 | P = 0.0230 | P = 0.0097 |

| | | Application of composition 3 outside of the invention | |
|---|---|---|---|
| T3h after cleansing | R = 0.063 | R = 0.079 | R = 0.086 |
| | P = 0.0440 | P = 0.0330 | P = 0.0260 |
| T6h after cleansing | R = 0.034 | R = 0.049 | R = 0.071 |
| | P = 0.2000 | P = 0.0700 | P = 0.0610 |

The values reported in table 15 (composition 3) above represent the means (n=30) of the beta diversity evaluated by the method detailed above. The greater the reduction in the value R, the lower the phylogenetic distance relative to T0 before cleansing, and the greater the recovery capacity of the bacterial community.

Based on the reference data at T0 before cleansing (bare skin), an increase in the R value is found, indicating an alteration and a distancing of the bacterial community. At D3 and D18, the value R has decreased, but remains significantly different relative to D0, again showing an alteration in the bacterial community and an absence of recovery at D0 and D3 and D18.

**[Table 16]**

| | D0 | D3 | D18 |
|---|---|---|---|
| T0 after cleansing | R = 0.071 | R = 0.097 | R = 0.100 |
| | P = 0.056 | P = 0.023 | P = 0.019 |

| | | Application of composition 2 according to the invention | |
|---|---|---|---|
| T3h after cleansing | R = 0.054 | R = 0.094 | R = 0.036 |
| | P = 0.078 | P = 0.052 | P = 0.180 |
| T6h after cleansing | R = 0.057 | R = 0.079 | R = 0.018 |
| | P = 0.074 | P = 0.068 | P = 0.420 |

The values reported in table 16 (composition 2) above represent the means (n=30) of the beta diversity evaluated by the method detailed above. The greater the reduction in the value R, the lower the phylogenetic distance relative to T0 before cleansing, and the greater the recovery capacity of the bacterial community.

Based on the reference data from before washing, no recovery is found at D0: the value of R is virtually unchanged between after washing (R= 0.071) and T6h (R= 0.057).

No recovery is observed at D3; the value of R is virtually unchanged between straight after washing (R= 0.097) and T6h (R= 0.079).

At D18 a rapid recovery is observed; the R value drops rapidly after cleansing (R= 0.100) and T3h (R= 0.036) and T6h (R= 0.018). A significant total recovery is observed at D18 from T3h.

### General conclusion:

Compositions 2 (according to the invention) and 3 (outside of the invention) enable a decrease in the pulling sensations.

Only composition 2 according to the invention, comprising a combination of oligosaccharides and mannose, enables a more rapid and more substantial recovery in the alpha and beta diversities following the use of an aggressive cleanser.

### Bibliographic references:

[1] Lai Y, Di Nardo A, Nakatsuji T, et al. Commensal bacteria regulate Toll-like receptor 3-dependent inflammation after skin injury. Nature Medicine 2009, 15: 1377-1382
[2] Nakatsuji T, Gallo RL. Antimicrobial Peptides: Old Molecules with New Ideas J Invest Dermatol 2012, 132, 3: 887-895
[3] Yuki T, Yoshida H, Akazawa Y et al. Activation of TLR2 Enhances Tight Junction Barrier in Epidermal Keratinocytes. J Immunol 2011, 187, 6, 3230-3237
[4] Byrd AL, Belkaid Y, Segre JA. The human skin microbiome. Nature Reviews Microbiology 2018, 16: 143-155
[5] Stacy A, Belkaid Y. Microbial guardians of skin health. Sciences 2019, 363, 6424: 227-228
[6] Linehan JL, Harrison OJ, Seong-JI et al. Non-classical Immunity Controls Microbiota Impact on Skin Immunity and Tissue Repair. Cell 2018, 172: 784-796
[7] Lee SE, Kim JM, Jeong SK, et al. Protease-activated receptor-2 mediates the expression of inflammatory cytokines, antimicrobial peptides, and matrix metalloproteinases in keratinocytes in response to Propionibacterium acnes. Archives of Dermatol Res 2010, 302, 10, 745-756
[8] linuma K, Sato T, Akimoto N, et al. Involvement of Propionibacterium acnes in the Augmentation of Lipogenesis in Hamster Sebaceous Glands In Vivo and In Vitro. J Invest Dermatol 2009, 129, 9: 2113-2119
[9] Allhorn M, Arve S, Brüggemann H et al. A novel enzyme with antioxidant capacity produced by the ubiquitous skin colonizer Propionibacterium acnes. Sci Rep. 2016; 6: 36412

## Claims

1. Cosmetic composition for topical application, comprising, in a physiologically acceptable medium:
- at least one oligosaccharide and/or polysaccharide, and
- at least one mannose monosaccharide,
in which said oligosaccharide and/or polysaccharide is in the form of a mixture comprising:
- at least one glucooligosaccharide (GOS), and
- at least one fructooligosaccharide (FOS),
wherein said glucooligosaccharide (GOS) is present in the composition in a concentration of between 0.01% and 10% by weight relative to the total weight of the composition ;
wherein said fructooligosaccharide (FOS) is present in the composition in a concentration of between 0.001% and 5% by weight relative to the total weight of the composition.

2. Composition according to the preceding claim, further comprising at least one ingredient chosen from silicone fatty substances such as silicone oils, gums and waxes; non-silicone fatty substances such as oils, pastes and waxes of plant, mineral, animal and/or synthetic origin; fatty acids having from 8 to 32 carbon atoms; synthetic esters and ethers, in particular of formula R₁COOR₂ and R¹OR² in which R¹ represents the residue of a fatty acid comprising from 8 to 29 carbon atoms, and R² represents a branched or unbranched hydrocarbon-based chain containing from 3 to 30 carbon atoms; linear or branched hydrocarbons of mineral or synthetic origin; fatty alcohols having from 8 to 26 carbon atoms; water; C2-C6 monoalcohols; glycols chosen from propylene glycol, butylene glycol, pentylene glycol; ketones; thickeners, emulsifiers, surfactants, gelling agents, fragrances, fillers, colourants, moisturizers, vitamins chosen from vitamin A, E, B3, polymers.

3. Composition according to any one of the preceding claims, in which said mannose monosaccharide is present in a concentration of from 0.01% to 20% by weight, preferably from 0.05% to 10% by weight, and most preferably from 0.1% to 5% by weight, relative to the total weight of the composition.

4. Composition according to any one of the preceding claims, further comprising at least one probiotic microorganism, in particular chosen from *Lactobacillus acidophilus, Lactobacillus alimentarius, Lactobacillus curvatus, Lactobacillus delbruckii subsp. Lactis, Lactobacillus gasseri, Lactobacillus johnsonii, Lactobacillus reuteri, Lactobacillus casei, Lactobacillus rhamnosus (Lactobacillus* GG), *Lactobacillus sake, Lactococcus lactis, Streptococcus thermophilus, Lactobacillus acidophilus, L. delbrueckii, L. helveticus, L. salivarius, L. curvatus, L. plantarum, L. sakei, L. brevis, L. buchneri, L. fermentum, L. reuteri, Lactobacillus bulgaricus, Lactobacillus longum, Lactobacillus lactis, Bifidobacterium longum* and mixtures thereof.

5. Composition according to Claim 4, in which said probiotic microorganism is present in a concentration of between 0.0001% and 10% by weight, preferably between 0.001% and 5% by weight, even more preferably between 0.001% and 1% by weight relative to the total weight of the composition.

6. Method of cosmetic, non-therapeutic treatment for caring for the skin and/or mucous membranes, comprising the application to the skin and/or to the mucous membranes, in particular having undergone external aggression, of the composition as defined in any one of Claims 1 to 5.

7. Non-therapeutic method according to Claim 6, for maintaining and/or restoring the balance of the bacterial flora of the skin and/or mucous membranes.

8. Non-therapeutic method according to Claim 6, for preventing and/or treating sensations of discomfort of sensitive skin chosen from itching, heating, or sensations of stinging, and/or of pulling.

9. Non-therapeutic method according to Claim 6, for preventing and/or treating detractions from the aesthetic qualities of the skin and/or mucous membranes, especially dry and/or rough skin.

10. Topical cosmetic, non-therapeutic use of at least oligosaccharide and/or polysaccharide as defined in claim 1 and of at least one mannose monosaccharide as defined in any one of claims 1 and 3 and optionally of at least one probiotic microorganism as defined in either of Claims 4 and 5 for caring for the skin, in particular skin which has undergone external aggression.

11. Non-therapeutic use according to Claim 10, for maintaining and/or restoring the balance of the bacterial flora of the skin and/or mucous membranes.

12. Non-therapeutic use according to Claim 10, for preventing and/or treating sensations of discomfort of sensitive skin chosen from itching, heating, or sensations of stinging, and/or of pulling.

13. Non-therapeutic use according to Claim 10, for preventing and/or treating detractions from the aesthetic qualities of the skin and/or mucous membranes, especially dry and/or rough skin.

## Patentansprüche

1. Kosmetische Zusammensetzung zur topischen Anwendung, umfassend in einem physiologisch verträglichen Medium:
- mindestens ein Oligosaccharid und/oder Polysaccharid, und
- mindestens ein Mannosemonosaccharid,
wobei das Oligosaccharid und/oder Polysaccharid in Form einer Mischung vorliegt, die Folgendes umfasst:
- mindestens ein Glucooligosaccharid (GOS), und
- mindestens ein Fructooligosaccharid (FOS),
wobei das Glucooligosaccharid (GOS) in der Zusammensetzung in einer Konzentration zwischen 0,01 bis 10 Gew.-% vorliegt, bezogen auf das Gesamtgewicht der Zusammensetzung;
wobei das Fructooligosaccharid (FOS) in der Zusammensetzung in einer Konzentration zwischen 0,001 bis 5 Gew.-% vorliegt, bezogen auf das Gesamtgewicht der Zusammensetzung.

2. Zusammensetzung nach dem vorhergehenden Anspruch, ferner umfassend mindestens einen Bestandteil ausgewählt aus Silikonfettsubstanzen, wie Silikonölen, -gummis und -wachsen; Nicht-Silikonfettsubstanzen, wie Ölen, Pasten und Wachsen pflanzlichen, mineralischen, tierischen und/oder synthetischen Ursprungs; Fettsäuren mit 8 bis 32 Kohlenstoffatomen; synthetischen Estern und Ethern, insbesondere der Formel R₁COOR₂ und R¹OR², worin R¹ für den Rest einer Fettsäure mit 8 bis 29 Kohlenstoffatomen steht und R² für eine verzweigte oder unverzweigte Kette auf Kohlenwasserstoffbasis mit 3 bis 30 Kohlenstoffatomen steht; linearen oder verzweigten Kohlenwasserstoffen mineralischen oder synthetischen Ursprungs; Fettalkoholen mit 8 bis 26 Kohlenstoffatomen; Wasser; C2-C6-Monoalkoholen; Glykolen ausgewählt aus Propylenglykol, Butylenglykol, Pentylenglykol; Ketonen; Verdickungsmitteln, Emulgatoren, Tensiden, Geliermitteln, Duftstoffen, Füllstoffen, Färbungsmitteln, Feuchthaltemitteln, Vitaminen ausgewählt aus Vitamin A, E, B3, Polymeren.

3. Zusammensetzung nach einem der vorhergehenden Ansprüche, wobei das Mannosemonosaccharid in einer Konzentration von 0,01 bis 20 Gew.-%, vorzugsweise 0,05 bis 10 Gew.-% und am meisten bevorzugt 0,1 bis 5 Gew.-% vorliegt, bezogen auf das Gesamtgewicht der Zusammensetzung.

4. Zusammensetzung nach einem der vorhergehenden Ansprüche, ferner umfassend mindestens einen probiotischen Mikroorganismus, insbesondere ausgewählt aus Lactobacillus acidophilus, Lactobacillus alimentarius, Lactobacillus curvatus, Lactobacillus delbruckii subsp. Lactis, Lactobacillus gasseri, Lactobacillus johnsonii, Lactobacillus reuteri, Lactobacillus casei, Lactobacillus rhamnosus (Lactobacillus GG), Lactobacillus sake, Lactococcus lactis, Streptococcus thermophilus, Lactobacillus acidophilus, L. delbrueckii, L. helveticus, L. salivarius, L. curvatus, L. plantarum, L. sakei, L. brevis, L. buchneri, L. fermentum, L. reuteri, Lactobacillus bulgaricus, Lactobacillus longum, Lactobacillus lactis, Bifidobacterium longum und Mischungen davon.

5. Zusammensetzung nach Anspruch 4, wobei der probiotische Mikroorganismus in einer Konzentration zwischen 0,0001 und 10 Gew.-%, vorzugsweise 0,001 bis 5 Gew.-%, noch bevorzugter zwischen 0,001 und 1 Gew.-% vorhanden ist, bezogen auf das Gesamtgewicht der Zusammensetzung.

6. Verfahren zur kosmetischen, nicht-therapeutischen Behandlung zur Pflege der Haut und/oder Schleimhäute, umfassend das Aufbringen der Zusammensetzung gemäß einem der Ansprüche 1 bis 5 auf die Haut und/oder die Schleimhäute, insbesondere die äußeren aggressiven Einflüssen ausgesetzt waren.

7. Nicht-therapeutisches Verfahren nach Anspruch 6 zur Bewahrung und/oder Wiederherstellung des Gleichgewichts der Bakterienflora der Haut und/oder Schleimhäute.

8. Nicht-therapeutisches Verfahren nach Anspruch 6 zur Vorbeugung und/oder Behandlung von Empfindungen von Unbehagen empfindlicher Haut ausgewählt aus Jucken, Hitzegefühl oder Empfindungen von Stechen und/oder von Ziehen.

9. Nicht-therapeutisches Verfahren nach Anspruch 6 zur Vorbeugung und/oder Behandlung von Beeinträchtigungen der ästhetischen Qualitäten der Haut und/oder Schleimhäute, insbesondere trockener und/oder rauer Haut.

10. Topische kosmetische, nicht therapeutische Verwendung von mindestens Oligosaccharid und/oder Polysaccharid wie in Anspruch 1 definiert und von mindestens einem Mannosemonosaccharid wie in einem der Ansprüche 1 und 3 definiert und gegebenenfalls von mindestens einem probiotischen Mikroorganismus wie in einem der Ansprüche 4 oder 5 definiert zur Pflege der Haut, insbesondere Haut, die äußeren aggressiven Einflüssen ausgesetzt war.

11. Nicht-therapeutische Verwendung nach Anspruch 10 zur Bewahrung und/oder Wiederherstellung des Gleichgewichts der Bakterienflora der Haut und/oder Schleimhäute.

12. Nicht-therapeutische Verwendung nach Anspruch 10 zur Vorbeugung und/oder Behandlung von Empfindungen von Unbehagen empfindlicher Haut, ausgewählt aus Jucken, Hitzegefühl oder Empfindungen von Stechen und/oder Ziehen.

13. Nicht-therapeutische Verwendung nach Anspruch 10 zur Vorbeugung und/oder Behandlung von Beeinträchtigungen der ästhetischen Qualitäten der Haut und/oder Schleimhäute, insbesondere trockener und/oder rauer Haut.

## Revendications

1. Composition cosmétique pour une application topique, comprenant dans un milieu physiologiquement acceptable :
- au moins un oligosaccharide et/ou polysaccharide, et
- au moins un monosaccharide de mannose,
dans laquelle ledit oligosaccharide et/ou polysaccharide est sous la forme d'un mélange comprenant :
- au moins un gluco-oligosaccharide (GOS), et
- au moins un fructo-oligosaccharide (FOS),
ledit gluco-oligosaccharide (GOS) étant présent dans la composition à une concentration comprise entre 0,01 % et 10 % en poids par rapport au poids total de la composition ;
ledit fructo-oligosaccharide (FOS) étant présent dans la composition à une concentration comprise entre 0,001 % et 5 % en poids par rapport au poids total de la composition.

2. Composition selon la revendication précédente, comprenant en outre au moins un composant choisi parmi les corps gras siliconés tels que les huiles, les gommes et les cires de silicone ; les corps gras non siliconés tels que les huiles, les pâtes et les cires **d'origine** végétale, minérale, animale et/ou synthétique ; les acides gras ayant de 8 à 32 atomes de carbone ; les esters et les éthers de synthèse, notamment de formules R₁COOR₂ et R¹OR², dans lesquelles R¹ représente le résidu **d'un** acide gras comprenant 8 à 29 atomes de carbone, et R² représente une chaîne hydrocarbonée, ramifiée ou non ramifiée, de 3 à 30 atomes de carbone ; les hydrocarbures linéaires ou ramifiés, d'origine minérale ou synthétique ; les alcools gras ayant de 8 à 26 atomes de carbone ; l'eau ; les monoalcools en C2-C6 ; les glycols choisis parmi le propylène glycol, le butylène glycol, le pentylène glycol ; les cétones ; les épaississants, les émulsifiants, les tensioactifs, les agents gélifiants, les parfums, les charges, les colorants, les agents hydratants, les vitamines choisies parmi les vitamines A, E, B3, les polymères.

3. Composition selon l'une quelconque des revendications précédentes, dans laquelle ledit monosaccharide de mannose est présent à une concentration de 0,01 % à 20 % en poids, de préférence de 0,05 % à 10 % en poids, et tout particulièrement de préférence de 0,1 % à 5 % en poids, par rapport au poids total de la composition.

4. Composition selon l'une quelconque des revendications précédentes, comprenant en outre au moins un microorganisme probiotique, en particulier choisi parmi Lactobacillus acidophilus, Lactobacillus alimentarius, Lactobacillus curvatus, Lactobacillus delbruckii subsp. Lactis, Lactobacillus gasseri, Lactobacillus johnsonii, Lactobacillus reuteri, Lactobacillus casei, Lactobacillus rhamnosus (Lactobacillus GG), Lactobacillus sake, Lactococcus lactis, Streptococcus thermophilus, Lactobacillus acidophilus, L. delbrueckii, L. helveticus, L. salivarius, L. curvatus, L. plantarum, L. sakei, L. brevis, L. buchneri, L. fermentum, L. reuteri, Lactobacillus bulgaricus, Lactobacillus longum, Lactobacillus lactis, Bifidobacterium longum, et des mélanges de ceux-ci.

5. Composition selon la revendication 4, dans laquelle ledit microorganisme probiotique est présente à une concentration comprise entre 0,0001 % et 10 % en poids, de préférence entre 0,001 % et 5 % en poids, encore plus préférentiellement entre 0,001 % et 1 % en poids, par rapport au poids total de la composition.

6. Méthode de traitement cosmétique, non thérapeutique de soin de la peau et/ou des muqueuses comprenant l'application sur la peau et/ou sur les muqueuses, en particulier ayant subi une agression externe, de la composition telle que définie dans l'une quelconque des revendications 1 à 5.

7. Méthode non thérapeutique selon la revendication 6, pour maintenir et/ou restaurer l'équilibre de la flore bactérienne de la peau et/ou des muqueuses.

8. Méthode non thérapeutique selon la revendication 6 pour prévenir et/ou traiter les sensations d'inconfort des peaux sensibles choisies parmi les démangeaisons, les échauffements, les sensations de picotement et/ou de tiraillement.

9. Méthode non thérapeutique selon la revendication 6, pour prévenir et/ou traiter les dégradations des aspects esthétiques de la peau et/ou des muqueuses, notamment les peaux sèches et/ou rugueuses.

10. Utilisation cosmétique, non thérapeutique, topique, d'au moins oligosaccharide et/ou polysaccharide tel que défini dans la revendication 1 et d'au moins un monosaccharide de mannose tel que défini dans l'une quelconque des revendications 1 et 3, et facultativement d'au moins un microorganisme probiotique tel que défini dans l'une des revendications 4 et 5, pour le soin de la peau, en particulier d'une peau ayant subi une agression externe.

11. Utilisation non thérapeutique selon la revendication 10 pour maintenir et/ou de restaurer l'équilibre de la flore bactérienne de la peau et/ou des muqueuses.

12. Utilisation non thérapeutique selon la revendication 10, pour prévenir et/ou traiter les sensations d'inconfort des peaux sensibles choisies parmi les démangeaisons, les échauffements, les sensations de picotement et/ou de tiraillement.

13. Utilisation non thérapeutique selon la revendication 10, pour prévenir et/ou traiter les dégradations des aspects esthétiques de la peau et/ou des muqueuses, notamment les peaux sèches et/ou rugueuses.
